# EUROPEAN PATENT SPECIFICATION

(11) **EP 2 305 831 B1**
(45) Date of publication and mention of the grant of the patent: **13.08.2014**
(21) Application number: 09770026.4
(22) Date of filing: 11.06.2009
(51) Int. Cl.: C12Q 1/02, C12Q 1/06

(54) **DILUTING SOLUTION FOR SUSPENSION FOR USE IN THE MEASUREMENT OF NUMBER OF LIVING MICROBIAL CELLS CONTAINED IN SAMPLE, AND METHOD FOR MEASUREMENT OF NUMBER OF LIVING MICROBIAL CELLS**
VERDÜNNUNGSLÖSUNG FÜR EINE SUSPENSION ZUR VERWENDUNG ZUR MESSUNG DER ANZAHL LEBENDER MIKROBIELLER ZELLEN IN EINER PROBE UND VERFAHREN ZUR MESSUNG DER ANZAHL LEBENDER MIKROBIELLER ZELLEN
SOLUTION DE DILUTION POUR UNE SUSPENSION DESTINÉE À ÊTRE UTILISÉE DANS LA MESURE DE LA QUANTITÉ DE CELLULES MICROBIENNES VIVANTES CONTENUES DANS UN ÉCHANTILLON, ET PROCÉDÉ DE MESURE DE LA QUANTITÉ DE CELLULES MICROBIENNES VIVANTES

(30) Priority: 24.06.2008 JP 2008164226
(43) Date of publication of application: 06.04.2011
(73) Proprietor: Morinaga Milk Industry Co., Ltd., Minato-ku Tokyo 108-8384 (JP)
(72) Inventor: MUTO Masamichi, Zama-shi Kanagawa 228-8583 (JP)
(74) Representative: Vossius & Partner
(86) International application number: PCT/JP2009/060701
(87) International publication number: WO 2009/157316

(56) References cited:
- JP-A- 6 038 795
- JP-A- H11 504 049
- JP-A- 2002 017 337
- US-A- 5 677 140
- WALKER K ET AL: "Rapid enumeration of Bifidobacterium lactis in milk powders using impedance", INTERNATIONAL DAIRY JOURNAL, ELSEVIER APPLIED SCIENCE, BARKING, GB, vol. 15, no. 2, 1 February 2005 (2005-02-01), pages 183-188, XP004706936, ISSN: 0958-6946, DOI: 10.1016/J.IDAIRYJ.2004.06.004
- ROTH B L ET AL: "BACTERIAL VIABILITY AND ANTIBIOTIC SUSCEPTIBILITY TESTING WITH SYTOX GREEN NUCLEIC ACID STAIN", APPLIED AND ENVIRONMENTAL MICROBIOLOGY, AMERICAN SOCIETY FOR MICROBIOLOGY, US, vol. 63, no. 6, 1 January 1997 (1997-01-01), pages 2421-2431, XP000929110, ISSN: 0099-2240
- FUMIAKI ABE*, HIROFUMI MIYAUCHI, AYAKO UCHIJIMA, TOMOKO YAESHIMA, KEIJI IWATSUKI: "Stability of bifidobacteria in powdered formula", INTERNATIONAL JOURNAL OF FOOD SCIENCE AND TECHNOLOGY, vol. 44, no. 4, 13 March 2009 (2009-03-13) , - 1 April 2009 (2009-04-01), pages 718-724, XP055004179, DOI: 10.1111/j.1365-2621.2008.01881.x
- WORKMAN, W. E. ET AL.: 'Microbial sterility testing of oil-formulated bovine somatotropin using Tween® 80 dispersion' JOURNAL OF PHARMACEUTICAL AND BIOMEDICAL ANALYSIS vol. 15, 1996, pages 193 - 200, XP008138804

## Description

### TECHNICAL FIELD

The present invention relates to a dilution buffer for suspension, which is used for generating a suspension buffer of a sample when measuring the viable cell count of a microorganism contained within the sample, and also relates to an enumeration method that uses the dilution buffer for suspension.

Priority is claimed on Japanese Patent Application No. 2008-164226, filed June 24, 2008,

### BACKGROUND ART

Bifidobacterium is widely known as one of the useful intestinal bacteria, and a multitude of publications exist relating to the physiological significance of this bacterium. It is very clear that bifidobacterium produces organic acids such as lactic acid and acetic acid within the intestine, and has effects in terms of suppressing the multiplication of harmful bacteria, producing vitamins, and activating immunity and the like (see Non-Patent Document 1). As a result, many food items have been developed that contain bifidobacteria, including yoghurts, cakes, drinks and health products and the like, with the aim of maintaining good health through the ingestion of bifidobacteria (see Non-Patent Document 2). Furthermore, infants who are fed with breast milk during infancy have superior levels of bifidobacteria, and therefore products such as powdered formula for infants or children containing bifidobacteria are also being developed in countries outside Japan.

For this variety of food items containing bifidobacteria, a measurement of the viable cell count is conducted to indicate the number of bifidobacteria contained within the food item.

In a widely used conventional method of measuring the viable cell count of a microorganism within a sample, a suspension buffer is first prepared containing the sample, the suspension buffer is serially diluted using a dilution buffer (such as a physiological saline solution), each of the resulting diluted samples is cultured using a culture method such as a pour plate method or a spread plate method, and the number of colonies formed on the culture medium is counted. In those cases where the sample is in a powdered form (such as a microorganism-containing bacterium powder or a powdered product such as a milk powder), the suspension buffer is prepared by adding the dilution buffer to the sample and stirring.

This conventional method poses no problems when used for direct measurement of the viable cell count within a bifidobacterium powder, but when measurement is conducted of the viable cell count of bifidobacteria added to a food item, there can be considerable variation in the measured values obtained. Furthermore, another problem arises in that these measured values tend to be lower than the viable cell count actually contained within the sample (the actual cell count). This problem is particularly marked when measuring the viable cell count of a bifidobacterium added to a powdered product such as a milk powder. For example, when the viable cell count of a bifidobacterium contained within a milk powder is measured using the above conventional method, the resulting measurement tends to be significantly lower than the viable cell count actually contained within the powder (the actual cell count) (for example, only 60 to 70% of the actual cell count).

In this manner, because the viable cell count of bifidobacteria added to various food items cannot be measured accurately, analysis organizations and the like have been seeking the development of a measurement method capable of yielding accurate measurements of the viable cell count.

Due to the circumstances described above, no official method exists for the measurement of the viable cell count of bifidobacteria within food items, although Non-Patent Document 3 discloses an enumeration method for bifidobacteria contained within fermented milk and lactobacillus beverages which is recommended by the Japanese Association of Fermented Milks and Fermented Milk Drinks. In this method, an anaerobic sample dilution buffer (hereafter referred to as "dilution buffer (A)") disclosed in "Standard methods of analysis in food safety regulation" is recommended as the dilution buffer. Further, Non-Patent Document 3 also discloses that physiological saline solution can also be used instead of the dilution buffer (A).

The dilution buffer (A) is a solution composed of KH₂PO₄: 4.5 g, Na₂HPO₄: 6.0 g, L-cysteine HCl·H₂O: 0.5 g, Tween 80: 0.5 g, agar: 1.0 g and pure water: 1,000 mL, and is widely used for the detection of anaerobic bacteria that exist within intestinal flora.

### PRIOR ART DOCUMENTS

### NON-PATENT DOCUMENTS

[Non-Patent Document 1] "Bifidus Research", written and edited by Tomotari Mitsuoka, chapter 1, pages 9 to 14, published by Japan Bifidus Foundation (1994)
[Non-Patent Document 2] "Bifidus Research", written and edited by Tomotari Mitsuoka, chapter 7, pages 267 and 282, published by Japan Bifidus Foundation (1994)
[Non-Patent Document 3] "Enumeration method for bifidobacterium within fermented milk and lactobacillus beverages", page 6, published by the Bifidobacterium Testing Methods Review Committee of the Japanese Association of Fermented Milks and Fermented Milk Drinks (2000).

### DISCLOSURE OF INVENTION

### PROBLEMS TO BE SOLVED BY THE INVENTION

However, in the case of powdered products, even if the above dilution buffer (A) or physiological saline solution is used as the dilution buffer, then in a similar manner to that described above, measurement of the viable cell count of bifidobacteria added to the powdered product such as milk powder still tends to be significantly lower than the actual cell count.

This low detection of the viable cell count is also problematic from an economic viewpoint, because when adding a bifidobacterium to a powdered product such as a powdered formula for infants or children, an amount of the bifidobacterium equivalent to two or more times the prescribed value must be added to the powdered product.

The present invention aims to address the problems outlined above, and has an object of providing a dilution buffer for suspension and an enumeration method, which, for the measurement of the viable cell count of a microorganism contained within a sample, and particularly a powdered product such as a milk powder, yield more accurate measurement values than have conventionally been obtainable.

### MEANS TO SOLVE THE PROBLEMS

As a result of intensive investigation, the inventors of the present invention discovered that by including at least a predetermined amount of polysorbates within the dilution buffer (dilution buffer for suspension) used when converting a sample containing a microorganism to a suspension buffer, the above object could be achieved, and they were therefore able to complete the present invention.

The present invention that achieves the object described above includes the following aspects.
[1] A dilution buffer for suspension, used for preparing a suspension buffer of a sample when conducting a measurement of the viable cell count of a microorganism contained within the sample, the dilution buffer containing polysorbates at a concentration of not less than 0.5%.
[2] The dilution buffer for suspension according to [1] above, wherein the concentration of the polysorbates is within a range from 0.5 to 30%.
[3] The dilution buffer for suspension according to [1] or [2] above, wherein the polysorbates are at least one polysorbate selected from the group consisting of Tween 40, Tween 60 and Tween 80.
[4] The dilution buffer for suspension according to any one of [1] to [3] above, wherein the microorganism is a bifidobacterium.
[5] An enumeration method for a microorganism contained within a sample, the method including: a step of preparing a suspension buffer of the sample using the dilution buffer for suspension according to any one of [1] to [4] above, and a step of measuring the viable cell count of the microorganism contained within the suspension buffer.
[6] The enumeration method according to [5] above, wherein the microorganism is a bifidobacterium.
[7] The enumeration method according to [5] or [6] above, wherein the sample is in a powdered form.
[8] The enumeration method according to [7] above, wherein the sample is a powdered formula for infants or children.

In the description and the claims of the present invention, concentration (%) values represent w/v (weight/volume) values.

Bifidobacterium that has been converted to a powdered form is termed "bifidobacterium powder", lactic acid bacteria other than bifidobacterium are termed "lactobacillus", and lactobacillus that has been converted to a powdered form is termed "lactobacillus powder". Further, the cells or bacterial cells of a microorganism may be referred to jointly as "bacterial cells".

In this description, "bifidobacterium" refers to a microorganism of the genus *Bifidobacterium*. The term "lactic acid bacterium" describes an anaerobic bacterium that produces lactic acid via fermentation.

### EFFECT OF THE INVENTION

The present invention is able to provide a dilution buffer for suspension and an enumeration method, which, for the measurement of the viable cell count of a microorganism contained within a sample, and particularly a powdered product such as a milk powder, yield more accurate measurement values than have conventionally been obtainable.

### BEST MODE FOR CARRYING OUT THE INVENTION

### <Dilution buffer for suspension>

A dilution buffer for suspension according to the present invention contains polysorbates at a concentration of not less than 0.5%.

The polysorbates are sorbitan fatty acid esters to which ethylene oxides have been bonded (namely, a polyoxyethylene sorbitan fatty acid ester). The number of ethylene oxide molecules bonded to the sorbitan fatty acid ester is typically approximately 20 molecules.

Sorbitan fatty acid esters are obtained by reacting sorbitol with a fatty acid, wherein the fatty acid is a compound represented by R-COOH in which R generally represents a saturated or unsaturated hydrocarbon group of 11 to 17 carbon atoms. Specific examples of the fatty acid include lauric acid, stearic acid, palmitic acid and oleic acid.

Specific examples of the polysorbates include Tween 20, Tween 40, Tween 60, Tween 65 and Tween 80.

Tween 20 is also called polysorbate 20 or polyoxyethylene sorbitan monolaurate.

Tween 40 is also called polysorbate 40 or polyoxyethylene sorbitan monopalmitate.

Tween 60 is also called polysorbate 60 or polyoxyethylene sorbitan monostearate.

Tween 65 is also called polysorbate 65 or polyoxyethylene sorbitan tristearate.

Tween 80 is also called polysorbate 80 or polyoxyethylene sorbitan monooleate.

Commercially available products may be used for any of these polysorbates.

The polysorbates used in the present invention are preferably at least one polysorbate selected from the group consisting of Tween 40, Tween 60 and Tween 80, as these polysorbates yield a superior effect for the present invention.

The concentration of the polysorbates within the dilution buffer for suspension is not less than 0.5%, and is preferably 1 % or greater. If the concentration of the polysorbates is less than 0.5%, then the effect of the present invention may be unobtainable.

Further, if the concentration of the polysorbates exceeds 30%, then the solubility tends to deteriorate, and therefore the concentration of the polysorbates within the dilution buffer for suspension is preferably within a range from 0.5 to 30%. If economic factors are taken into consideration, then the concentration is preferably not more than 2%.

The dilution buffer for suspension according to the present invention may also include other components besides the polysorbates, provided these other components do not impair the effects of the present invention.

There are no particular limitations on these other components, and the types of components typically added to dilution buffers may be used. Specific examples include sodium chloride, potassium dihydrogen phosphate, disodium hydrogen phosphate, L-cysteine hydrochloride monohydrate, agar, soy peptide, sodium L-ascorbate, peptone, yeast extract and Ringer's solution.

The dilution buffer for suspension according to the present invention can be prepared by mixing the polysorbates and any optional components with water.

The dilution buffer for suspension according to the present invention described above is used for preparing a suspension buffer of a sample when conducting a measurement of the viable cell count of a microorganism contained within the sample. Detailed descriptions of the sample and the enumeration method used for measuring the viable cell count are presented below within the description of the enumeration method according to the present invention.

### <Enumeration method>

The enumeration method according to the present invention includes: preparing a suspension buffer of a sample containing a microorganism using the aforementioned dilution buffer for suspension according to the present invention, and measuring the viable cell count of the microorganism contained within the suspension buffer.

Examples of the microorganism include bifidobacteria and lactic acid bacteria. Of these, a bifidobacterium is preferred.

There are no particular limitations on the bifidobacterium, and specific examples include *Bifidobacterium longum* subsp. *longum, Bifidobacterium longum* subsp. *infantis, Bifidobacterium adolescentis, Bifidobacterium breve, Bifidobacterium bifidum* and *Bifidobacterium animalis* subsp. *lactis*. All of these bacterial strains are either available commercially, or can be readily obtained from a microorganism depository. Furthermore, powdered products and health food items containing these bacterial strains can also be purchased easily.

Examples of the lactic acid bacteria include *Lactobacillus, Lactococcus, Enterococcus* and *Streptococcus* and the like.

There are no particular restrictions on the types of samples which can be measured for viable cell count using the present invention, provided the sample contains a microorganism, although if consideration is given to the ease of preparation of the suspension buffer, then a powdered sample is preferred.

Specific examples of powdered samples include products prepared by blending bacterial cells of a microorganism such as a bifidobacterium or lactic acid bacterium into a powdered product. Specific examples of the powdered product include milk powders such as powdered formula for infants or children and milk powder for pregnant women. Furthermore, a solid sample containing added bacterial cells of a microorganism may be subjected to a crushing treatment to obtain a powdered sample.

In the present invention, the sample is preferably a powdered formula for infants or children, and particularly a powdered formula for infants or children containing a bifidobacterium.

With the exception of using the dilution buffer for suspension according to the present invention when preparing the suspension, the suspension buffer of the sample may be prepared using a conventional method of preparing suspension buffers. For example, in the case where the sample is in a powdered form, the suspension buffer can be prepared by mixing and stirring the sample and the dilution buffer for suspension according to the present invention.

Measurement of the viable cell count of a microorganism contained within a suspension buffer can be performed using a culture method in which culturing is performed on an agar medium (namely, a solid culture method).

Specifically, this culture method may be performed by serially diluting the suspension buffer, culturing each of the resulting diluted samples on an agar medium, and then counting the number of colonies that are formed. The number of colonies formed corresponds with the viable cell count of the microorganism contained within the diluted sample cultured on the agar medium. Accordingly, the viable cell count for the microorganism can be determined from the value for the number of colonies and the dilution ratio. Based on the viable cell count within the suspension buffer determined in this manner, and the amount of the sample contained within the suspension buffer, the viable cell count (/g) of the microorganism within the sample can be determined.

In the above method, a dilution buffer (secondary dilution buffer) is used for performing the serial dilution of the suspension buffer. In the present invention, the dilution buffer used when preparing the suspension buffer employs the dilution buffer for suspension according to the present invention, but there are no particular restrictions on the nature of the secondary dilution buffer. Examples of materials that may be used as the secondary dilution buffer include physiological saline solution and the dilution buffer (A) described above (the anaerobic sample dilution buffer disclosed in "Standard methods of analysis in food safety regulation").

Specific examples of the culture method include the pour plate method and the spread plate method. The pour plate method is a method in which an appropriately diluted suspension buffer is mixed with a heated and melted agar medium, and the mixture is then cooled, solidified and cultured. The spread plate method is a method in which an appropriately diluted suspension buffer is smeared across the top of an agar medium and then cultured.

In terms of the variety of agar medium used in the culture method, and the culture conditions (such as the culture temperature and culture time), conventional culture conditions may be employed in accordance with the nature of the microorganism being measured, and the agar medium may be any medium on which the microorganism being measured is able to be grown. Examples of agar media on which a bifidobacterium is able to be grown include reinforced clostridial agar medium and TOS propionate agar medium. Further, in the case of a bifidobacterium, the count of the number of colonies is usually conducted following anaerobic culturing for 72 hours at 37°C.

### EXAMPLES

A more detailed description of the present invention is presented below based on a series of test examples and examples, although the present invention is in no way limited by the following examples.

### <Test example 1>

In this test, a bifidobacterium powder was triturated into a milk powder, and the viable cell count was then measured to ascertain the reduction in cell count caused by the trituration.

### 1) Preparation of test samples:

As the bifidobacterium powder (raw material powder), a BB536 powder (manufactured by Morinaga Milk Industry Co., Ltd., viable cell count: 8.1 x 10¹⁰/g) was prepared.

The above raw material powder was mixed with a Morinaga follow-up milk "Chil-mil" (manufactured by Morinaga Milk Industry Co., Ltd.) in a ratio of 1:1000 (w/w) (namely, a 1,000 fold trituration), thus forming a triturated sample.

In the following description, the value obtained by dividing the viable cell count (actual cell count) of the raw material powder by 1,000 was used as the actual cell count for the triturated sample.

**[Table 1]**

| Test sample | Bacterium powder | Trituration ratio | Actual cell count |
|---|---|---|---|
| Raw material powder | BB536 powder | - | 8.1 × 10¹⁰/g |
| Triturated sample | BB536 powder | 1000-fold trituration (Chil-mil) | 8.1 × 10⁷ /g |

### 2) Preparation of dilution buffers for suspension:

Dilution buffers for suspension were prepared by adding sufficient pure water to each of the groups of components listed below in Table 2 to make a total volume of 1,000 ml, and the resulting mixtures were then stirred, yielding a physiological saline solution, a dilution buffer (A), and a 1% Tween 80-containing dilution buffer. Each of these dilution buffers for suspension was sterilized for 15 minutes at 121 °C in an autoclave.

**[Table 2]**

| Component | Physiological saline solution | Dilution buffer (A) | 1% Tween 80-containing dilution buffer |
|---|---|---|---|
| Sodium chloride | 8.5 g | | |
| Potassium dihydrogen phosphate | | 4.5 g | 4.5 g |
| Disodium hydrogen phosphate | | 6.0 g | 6.0 g |
| L-cysteine hydrochloride monohydrate | | 0.5 g | 0.5 g |
| Tween 80 | | 0.5 g | 10.0 g |
| Agar | | 1.0 g | |

### 3) Measurement of viable cell count by culture method (pour plate method):

A predetermined amount of the test sample (either 5 g of the triturated sample or 1 g of the raw material powder) was weighed, a sufficient amount of a dilution buffer for suspension (the physiological saline solution, dilution buffer (A), or 1% Tween 80-containing dilution buffer) was added to bring the total mass to 100 g (namely, 95 g per 5 g of the triturated sample (20-fold dilution), or 99g per 1 g of the raw material powder (100-fold dilution)), and the resulting mixture was stirred vigorously, thus completing preparation of a suspension buffer (samples 1-1 to 1-5).

Subsequently, using the physiological saline solution as a secondary dilution buffer, each of the prepared suspension buffers was diluted (one 100-fold dilution and one 10-fold dilution, totaling a 1,000-fold dilution), and the resulting dilution product was spread onto a plate together with a pre-melted reinforced clostridial medium agar (manufactured by Oxoid Ltd.) using the pour plate method. Following solidification of the agar, anaerobic culturing was conducted for 3 days at 37°C. Following the culturing, the number of colonies that had grown on the medium was counted and analyzed, thereby determining a measured value for the viable cell count within each of the test samples. The results are shown below in Table 3.

**[Table 3]**

| | Test sample [actual cell count] | Dilution buffer for suspension | Measured value for viable cell' count [ratio relative to actual cell count] |
|---|---|---|---|
| Sample 1-1 | Raw material powder [8.1 × 10¹⁰ /g] | Dilution buffer (A) | 7.7 × 10 ¹⁰ /g [95%] |
| Sample 1-2 | | 1% Tween 80-containing dilution buffer | 8.3 × 10¹⁰ /g [102%] |
| Sample 1-3 | Triturated sample [8.1 × 10⁷ /g] | Physiological saline solution | 5.6 × 10⁷ /g [69%] |
| Sample 1-4 | | Dilution buffer (A) | 5.9 × 10⁷ /g [73%] |
| Sample 1-5 | | 1% Tween 80-containing dilution buffer | 8.4 × 10⁷ /g [104%] |

As is evident from the above results, the measured values for the viable cell count in samples 1-1 and 1-2, in which the viable cell count was measured for the raw material BB536 powder, were from 95 to 102% of the actual cell count, and regardless of whether the dilution buffer (A) or the 1% Tween 80-containing dilution buffer was used as the dilution buffer for suspension, an accurate value that was close to the actual cell count was obtained.

On the other hand, in the case of the triturated sample prepared by a 1000-fold trituration of the BB536 powder with Chil-mil, the measured value for the viable cell count in the sample 1-3 wherein the suspension was prepared using the physiological saline solution was 5.6 × 10⁷ /g, indicating that only 69% of the actual cell count was detected. In a similar manner, in the sample 1-4 wherein the suspension was prepared using the dilution buffer (A), the measured value for the viable cell count was 5.9 × 10⁷ /g, indicating that only 73% of the actual cell count was detected.

In contrast, in the sample 1-5, wherein the suspension was prepared using the 1% Tween 80-containing dilution buffer, the measured value for the viable cell count within the triturated sample was 8.4 × 10⁷ /g, which represents an accurate value that was close to the actual cell count.

As described above, when measuring the viable cell count of a bacterium contained within a milk powder, if a physiological saline solution or the dilution buffer (A) is used as the dilution buffer for suspension, then the detected viable cell count is only 69 to 73% of the actual cell count, which represents a significant reduction and indicates that an accurate measurement of the viable cell count is impossible. In contrast, if a 1% Tween 80-containing dilution buffer is used as the dilution buffer for suspension, then even when the viable cell count of a bacterium contained within a milk powder is measured, the viable cell count can be measured as accurately as the case where the viable cell count of the raw material bacterium powder is measured directly.

### <Text example 2>

This test was performed to investigate the effect of the composition of the dilution buffer for suspension on the cell count reduction phenomenon observed in test example 1.

### 1) Preparation of dilution buffers for suspension:

Dilution buffers for suspension 1 to 4 were prepared by mixing 4.5 g of potassium dihydrogen phosphate, 6.0 g of disodium hydrogen phosphate, 0.5 g of L-cysteine hydrochloride monohydrate, one of component-1 to component-4 listed below in Table 4, and pure water. In each case, sufficient pure water was added to make the dilution buffer for suspension up to a total volume of 1,000 ml. Each of these dilution buffers for suspension was sterilized for 15 minutes at 121 °C in an autoclave.

In Table 4, the abbreviation "RCM medium" represents a reinforced clostridial medium (manufactured by Oxoid Ltd.). As the soy peptide, a product manufactured by Becton, Dickson and Company was used.

**[Table 4]**

| | Tween 80 | RCM medium | Soy peptide | Sodium L-ascorbate |
|---|---|---|---|---|
| Component-1 | 10.0 g | | | |
| Component-2 | 0.5 g | 20.0 g | | |
| Component-3 | 0.5 g | | 20.0 g | |
| Component-4 | 0.5 g | | | 20.0 g |

### 2) Test method:

As the test sample, the triturated sample prepared in test example 1 (namely, the 1000-fold triturated sample of BB536 powder) was used, and with the exception of using the dilution buffer (A) and the dilution buffers for suspension 1 to 4 as the dilution buffer for suspension, the viable cell counts were measured by culture method (pour plate method) in the same manner as test example 1.

The results are shown in Table 5.

**[Table 5]**

| | Dilution buffer for suspension | | Measured value for viable cell count [ratio relative to actual cell count] |
|---|---|---|---|
| | | Tween 80 concentration | |
| Sample 2-1 | Dilution buffer (A) | 0.05% | 5.9 × 10⁷ /g [73%] |
| Sample 2-2 | Dilution buffer for suspension 1 (Component-1) | 1.0% | 8.4 × 10⁷/g [104%] |
| Sample 2-3 | Dilution buffer for suspension 2 (Component-2) | 0.05% | 5.7 × 10⁷/g [70%] |
| Sample 2-4 | Dilution buffer for suspension 3 (Component-3) | 0,05% | 6.1 × 10⁷/g [75%] |
| Sample 2-5 | Dilution buffer for suspension 4 (Component-4) | 0.05% | 6.3 × 10⁷ /g [78%] |

As listed in Table 5, in the sample 2-1 in which the dilution buffer (A) containing 0.05% of Tween 80 was used as the dilution buffer for suspension, the measured value for the viable cell count was 5.9 × 10⁷ /g (73% of the actual cell count).

In contrast, in the sample 2-2 which used the dilution buffer for suspension 1 containing 1% of Tween 80, the measured value for the viable cell count was 8.4x 10⁷ /g (104% of the actual cell count), which represents an accurate value closer to the actual cell count than that observed for the sample 2-1.

In the samples 2-3 to 2-5, which used the dilution buffers for suspension 2 to 4, each of which had a Tween 80 concentration of 0.05%, but which also contained added RCM medium, soy peptide or sodium L-ascorbate respectively, the measured values for the viable cell count were from 70 to 78% of the actual cell count. Although two of these results were marginally closer to the actual cell count than that observed for the sample 2-1, a result that was as accurate as that obtained for the sample 2-2 was unobtainable.

From the above results it is clear that for a sample prepared by triturating a bacterium powder with a milk powder, using a dilution buffer for suspension containing 1% of Tween 80 enables the viable cell count to be measured more accurately.

### <Text example 3>

This test was performed to determine the ideal concentration range for Tween 80 within the dilution buffer for suspension, and to investigate the cell count when polysorbates other than Tween 80 were added.

### 1) Preparation of dilution buffers for suspension:

Dilution buffers for suspension 5 to 19 were prepared by mixing 4.5 g of potassium dihydrogen phosphate, 6.0 g of disodium hydrogen phosphate, 0.5 g of L-cysteine hydrochloride monohydrate, one of component-5 to component-19 listed below in Table 6, and pure water. In each case, sufficient pure water was added to make the dilution buffer for suspension up to a total volume of 1,000 ml. Each of these dilution buffers for suspension was sterilized for 15 minutes at 121°C in an autoclave.

The dilution buffers for suspension 5 to 15 represent buffers in which the Tween 80 concentration was varied from 0.2% to 30% in a stepwise manner, whereas the dilution buffers for suspension 16 to 19 contained 1.0% of Tween 20, Tween 40, Tween 60 and Tween 65 respectively.

**[Table 6]**

| | Tween 80 | Tween 20 | Tween 40 | Tween 60 | Tween 65 |
|---|---|---|---|---|---|
| Component-5 | 2.0 g | | | | |
| Component-6 | 5.0 g | | | | |
| Component-7 | 10.0 g | | | | |
| Component-8 | 20.0 g | | | | |
| Component-9 | 40.0 g | | | | |
| Component-10 | 60.0 g | | | | |
| Component-11 | 80.0 g | | | | |
| Component-12 | 100 g | | | | |
| Component-13 | 150 g | | | | |
| Component-14 | 200 g | | | | |
| Component-15 | 300 g | | | | |
| Component-16 | | 10.0 g | | | |
| Component-17 | | | 10.0 g | | |
| Component-18 | | | | 10.0 g | |
| Component-19 | | | | | 10.0 g |

### 2) Test method:

As the test sample, the triturated sample prepared in test example 1 (namely, the 1000-fold triturated sample of BB536 powder) was used, and with the exception of using the dilution buffer (A) and the dilution buffers for suspension 5 to 19 as the dilution buffer for suspension, the viable cell counts were measured by culture method (pour plate method) in the same manner as test example 1. The results are shown in Table 7.

**[Table 7]**

| | Dilution buffer for suspension | | | Measured value for viable cell count [ratio relative to actual cell count] |
|---|---|---|---|---|
| | | Polysorbates | Concentration (%) | |
| Sample 3-1 | Dilution buffer (A) | Tween 80 | 0.05 | 5.9 × 10⁷ /g [73%] |
| Sample 3-2 | Dilution buffer for suspension 5 (Component-5) | Tween 80 | 0.2 | 5.5 × 10⁷/g [68%] |
| Sample 3-3 | Dilution buffer for suspension 6 (Component-6) | Tween 80 | 0.5 | 7.0 × 10⁷ /g [86%] |
| Sample 3-4 | Dilution buffer for suspension 7 (Component-7) | Tween 80 | 1.0 | 8.4 × 10⁷/g [104%] |
| Sample 3-5 | Dilution buffer for suspension 8 (Component-8) | Tween 80 | 2.0 | 7.8 × 10⁷/g [96%] |
| Sample 3-6 | Dilution buffer for suspension 9 (Component-9) | Tween 80 | 4.0 | 8.0 × 10⁷/g [99%] |
| Sample 3-7 | Dilution buffer for suspension 10 (Component-10) | Tween 80 | 6.0 | 8.1 × /g [100%] |
| Sample 3-8 | Dilution buffer for suspension 11 (Component- 11) | Tween 80 | 8.0 | 7.4 × 10⁷/g [91%] |
| Sample 3-9 | Dilution buffer for suspension 12 (Component-12) | Tween 80 | 10 | 8.1 × 10⁷/g [100%] |
| Sample 3-10 | Dilution buffer for suspension 13 (Component-13) | Tween 80 | 15 | 8.7 × 10⁷/g [107%] |
| Sample 3-11 | Dilution buffer for suspension 14 (Component-14) | Tween 80 | 20 | 8.2 ×10⁷/g [101%] |
| Sample 3-12 | Dilution buffer for suspension 15 (Component-15) | Tween 80 | 30 | 8.2 × 10⁷/g [101%] |
| Sample 3-13 | Dilution buffer for suspension 16 (Component-16) | Tween 20 | 1.0 | 7.1 × 10⁷/g [88%] |
| Sample 3-14 | Dilution buffer for suspension 17 (Component-17) | Tween 40 | 1.0 | 7.9 × 10⁷ /g [98%] |
| Sample 3-15 | Dilution buffer for suspension 18 (Component-18) | Tween 60 | 1.0 | 8.2 × 10⁷ /g [101%] |
| Sample 3-16 | Dilution buffer for suspension 19 (Component-19) | Tween 65 | 1.0 | 6.9 × 10⁷ /g [85%] |

As listed in Table 7, in the sample 3-1 in which the dilution buffer (A) containing 0.05% of Tween 80 was used as the dilution buffer for suspension, the measured value for the viable cell count was 5.9 × 10⁷ /g (73% of the actual cell count). Further, in the sample 3-2 which used the dilution buffer for suspension 5 containing 0.2% of Tween 80, the measured value for the viable cell count was 5.5 × 10⁷ /g (68% of the actual cell count).

In contrast, in the samples 3-3 to 3-12, which used the dilution buffers for suspension 6 to 15 in which the concentration of Tween 80 was within a range from 0.5 to 30%, the measured values for the viable cell count were within a range from 86 to 107% of the actual cell count, indicating that accurate values close to the actual cell count were able to be obtained. In particular, in the samples 3-4 to 3-12, which used the dilution buffers for suspension 7 to 15 in which the concentration of Tween 80 was within a range from 1.0 to 30%, the measured values for the viable cell count (91% to 107%) were within a margin of error of ±9% of the actual cell count.

Furthermore, for the samples 3-13 to 3-16, which used the dilution buffers for suspension 16 to 19 containing 1.0% of the alternative polysorbates Tween 20, Tween 40, Tween 60 and Tween 65 respectively, the measured values for the viable cell count were 88%, 98%, 101% and 85% respectively of the actual cell count. Of these, the samples 3-14 and 3-15 containing Tween 40 and Tween 60 respectively yielded particularly accurate measured values.

From the above results it is clear that when measuring the viable cell count of a sample prepared by triturating a bacterium powder with a milk powder, using a dilution buffer for suspension containing from 0.5 to 30% of Tween 80 yields an accurate measured value close to the actual cell count, and moreover, a similar effect can be achieved by using other polysorbates (and particularly Tween 40 and Tween 60).

### <Example 1>

19 varieties of bifidobacterium-containing food items containing either a powdered formula for infants or children or a milk powder for pregnant women were prepared. For each of these food items, the product name, company name, and where recorded the variety of bifidobacterium are listed below in Table 8. All of these food items are commercially available items, and are readily available.

Of the companies listed in Table 8, "Dairyland" represents PT Dairyland Indonesia, "Morinaga^{a}" represents Morinaga Milk Industry Co., Ltd., "Morinaga^{b}" PT Kalbe Morinaga Indonesia, and "Morinaga^{c}" represents PT Sanghiang Perkasa. Further, "Abbott" represents P.T. Abbott Indonesia, and "Yili" represents Inner Mongolia Yili Industrial Group Co., Ltd.

In Table 8, *B. lactis* represents *Bifidobacterium animalis* subsp. *lactis,* and *B. longum* represents *Bifidobacterium longum* subsp. *longum.*

A PCR (polymerase chain reaction) method was used to confirm the variety of bifidobacterium contained within each product. The results are shown in Table 8.

As the dilution buffer for suspension, the three buffers used in test example 1, namely a physiological saline solution, the dilution buffer (A), and a 1% Tween 80-containing dilution buffer, were used. Measurement of the viable cell count of the bifidobacterium contained within each of the products was conducted using the procedure described below.

Each product was converted to a suspension using the dilution buffer for suspension. Following appropriate dilution (1 to 10⁶-fold dilution depending on the cell count) of the resulting suspension buffer with a physiological saline solution, a TOS propionate agar medium (manufactured by Eiken Chemical Co., Ltd.) was used to perform anaerobic culturing for 3 days at 37°C using the pour plate method. Following culturing, the number of colonies was counted.

The same operation was performed 3 times (with 3 Petri dishes used per product), and based on the results, viable cell counts (/g) for each of the 19 products were obtained using the physiological saline solution, the dilution buffer (A), and the 1% Tween 80-containing dilution buffer. The results are shown in Table 8. Prior to counting of the number of colonies, the bacteria of a representative colony were inspected under a microscope to confirm they were bifidobacteria.

Further, statistical analysis was performed on the counted number of colonies (the colony count). The statistical analysis was conducted using the software JMP (version 5.1.1, manufactured by SAS Institute Inc.), and a Tukey HSD test was performed via a two-way analysis of variance with the dilution buffer for suspension used and the product used acting as the two variables. The results are shown in Table 9.

**[Table 8]**

| Product name (common name) | Company name | Bifidobacterium variety | | Viable cell count | | | |
|---|---|---|---|---|---|---|---|
| | | Listed | PCR result | Physiological saline solution | Dilution buffer (A) | 1% Tween 80-containing dilution buffer | Units(g) |
| Prestine 1+ | Dairyland | *B. bifidum* | *B. bifidum* | 3.7±1.6 | 2.2±0,8 | 3.0±3.3 | × 10⁴ |
| Bifidus for infants | Morinaga^{a} | *B. breve* | *B. breve* | 1.5±0.1 | 1.6±0.1 | 1.9±0.1 | × 10¹⁰ |
| Pediasure complete | Abbott | - | *B. lactis* | 11.0±4.7 | 5.6±3.7 | 7.0±3.3 | × 10³ |
| Yili infant milk formula | Yili | - | *B. lactic* | 7.6±2.9 | 13.5±3.5 | 12.3±2.6 | × 10⁵ |
| Yili pregnant milk powder | Yili | - | *B. lactic* | 11.5±1.9 | 9.6±0.9 | 16.0±4.3 | × 10⁴ |
| Product A | Company a | - | *B. lactis* | 8.7±0.9 | 10.1±1.6 | 13.4±1.0 | × 10⁶ |
| Product B | Company a | - | *B. lactis* | 4.9±0.5 | 4.3±0.5 | 5.2±0.4 | × 10⁶ |
| Product C | Company b | - | *B. lactis* | 3.7±0.5 | 5.4±1.4 | 7.7±0.9 | × 10⁶ |
| Product D | Company c | *B. lactis* | *B. lactis* | 1.2±0.1 | 1.5±0.1 | 1.3±0.1 | × 10⁷ |
| Product E | Company b | - | *B. lactis* | 1.3±0.2 | 1.6±0.2 | 1.5±0.1 | × 10⁷ |
| Product F | Company b | - | *B. lactis* | 2.6±0.4 | 2.2±0.2 | 3.6±0.3 | × 10⁶ |
| Product G | Company d | - | *B. longum* | 5.1±1.8 | 7.4±2.5 | 11.0±1.2 | × 10⁶ |
| Product H | Company d | - | *B. longum* | 4.7±0.7 | 5.4±1.8 | 6.4±1.7 | × 10⁶ |
| Product I | Company d | - | *B. longum* | 6.8±1.2 | 9.2±1.6 | 12.4±2.6 | × 10⁶ |
| Product J | Company d | - | *B. longum* | 8.0±1.4 | 11.4±2.5 | 14.1±1.8 | × 10⁶ |
| Product K | Company d | - | *B. lorrgtrm* | 9.2±2.1 | 9.8±1.3 | 12.6±1.6 | × 10⁶ |
| Product L | Company d | - | *B*. *longum* | 1.4±0.5 | 3.1±0.4 | 2.9±0.3 | × 10⁶ |
| Morinaga Chil-kid Platinum | Morinaga | *B. longum* | *B. longum* | 1.8±0.3 | 2.1±0.1 | 2.3±0.1 | × 10⁷ |
| | | *B. breve* | *B. breve* | | | | |
| Morinaga Chil-school Platinum | Morinagga^{c} | *B. longum* | *B. longum* | 1.7±0.2 | 1.8±0.2 | 2.2±0.3 | × 10⁷ |
| | | *B. breve* | *B. breve* | | | | |

| | | | | | | | |
|---|---|---|---|---|---|---|---|
| *B. lactis: Bifidobacterium animalis* subsp. *lactis* *B. longum: Bifidobacterium longum* subsp. *longum* | | | | | | | |

**[Table 9]**

| | Physiological saline solution | Dilution buffer (A) | 1% Tween 80-containing dilution buffer |
|---|---|---|---|
| Value of least mean square of colony count (%) | 100 | 115 | 139* |
| Frequency with which highest viable cell count was obtained | 2/19 | 4/19 | 13/19 |

| | | | |
|---|---|---|---|
| * Statistically significant difference (relative to the physiological saline solution and dilution buffer (A)) | | | |

As shown in Table 9, for the 19 different varieties of commercially available products, if the least mean square of the colony count was deemed to be 100% for the samples that used the physiological saline solution, then the least mean square of the colony count was 115% for the dilution buffer (A), and 139% for the 1% Tween 80-containing dilution buffer. These results indicate that by using the 1% Tween 80-containing dilution buffer, a statistically significantly higher value is obtained when measuring the viable cell count. Further, of the products used, it was found that Morinaga Chil-kid and Morinaga Chil-school yielded measured values for the viable cell count that were substantially equal to the actual cell count blended into the product. These results confirmed that by using the 1% Tween 80-containing dilution buffer, the viable cell count could be measured more accurately than when using a physiological saline solution or the dilution buffer (A).

Further, when the 1% Tween 80-containing dilution buffer was used, the measured value for the viable cell count was highest for 13 of the 19 products. These results indicate that by using the 1% Tween 80-containing dilution buffer, the bifidobacterium cell count within a product can be measured in a stable manner.

As described in detail above, according to the present invention, by using a dilution buffer containing polysorbates at a concentration of not less than 0.5% as the dilution buffer for suspension when using a culture method to measure the viable cell count of a bifidobacterium powder triturated with a powdered formula for infants or children, the viable cell count of the bifidobacterium within the powdered formula for infants or children can be measured accurately.

### INDUSTRIAL APPLICABILITY

The present invention is able to provide a dilution buffer for suspension and an enumeration method, which, for the measurement of the viable cell count of a microorganism contained within a sample, and particularly a powdered product such as a milk powder, yield more accurate measurement values than have conventionally been obtainable, and is therefore useful in fields such as the production of food items.

## Claims

1. Use of a dilution buffer for suspension, for preparing a suspension buffer of a sample when conducting a measurement of a viable cell count of a microorganism contained within said sample, said dilution buffer comprising polysorbates at a concentration of not less than 0.5% and said microorganism is a bifidobacterium.

2. Use of the dilution buffer for suspension according to claim 1, wherein a concentration of said polysorbates is within a range from 0.5 to 30%.

3. Use of the dilution buffer for suspension according to claim 1 or claim 2, wherein said polysorbates are at least one polysorbate selected from the group consisting of Tween 40, Tween 60 and Tween 80.

4. An enumeration method for a microorganism contained within a sample, said method comprising:
a step of preparing a suspension buffer of said sample using the dilution buffer for suspension according to any one of claims 1 to 3, and
a step of measuring a viable cell count of said microorganism contained within said suspension buffer, wherein
said microorganism is a bifidobacterium.

5. The enumeration method according to claim 4, wherein said sample is in a powdered form.

6. The enumeration method according to claim 5, wherein said sample is a powdered formula for infants or children.

## Patentansprüche

1. Verwendung eines Verdünnungspuffers für eine Suspension zur Herstellung eines Suspensionspuffers einer Probe bei der Durchführung einer Messung einer Anzahl von lebenden Zellen eines in der Probe enthaltenen Mikroorganismus, wobei der Verdünnungspuffer Polysorbate in einer Konzentration von nicht weniger als 0,5 % umfasst und der Mikroorganismus ein Bifidobacterium ist.

2. Verwendung des Verdünnungspuffers für eine Suspension nach Anspruch 1, wobei eine Konzentration der Polysorbate in einem Bereich von 0,5 bis 30 % liegt.

3. Verwendung des Verdünnungspuffers für eine Suspension nach Anspruch 1 oder Anspruch 2, wobei die Polysorbate mindestens ein Polysorbat sind, ausgewählt aus der Gruppe bestehend aus Tween 40, Tween 60 und Tween 80.

4. Ein Zählverfahren für einen in einer Probe enthaltenen Mikroorganismus, wobei das Verfahren umfasst:
einen Schritt des Herstellens eines Suspensionspuffers der Probe unter Verwendung des Verdünnungspuffers für eine Suspension nach einem der Ansprüche 1 bis 3, und
einen Schritt des Messens einer Anzahl von lebenden Zellen des in dem Suspensionspuffer enthaltenen Mikroorganismus, wobei
der Mikroorganismus ein Bifidobacterium ist.

5. Das Zählverfahren nach Anspruch 4, wobei die Probe in einer Pulverform vorliegt.

6. Das Zählverfahren nach Anspruch 5, wobei die Probe in einer Pulverformel für Säuglinge oder Kinder vorliegt.

## Revendications

1. Utilisation d'un tampon de dilution pour suspension, pour la préparation d'un tampon de suspension d'un échantillon lors de la réalisation d'une mesure d'un nombre de cellules viables d'un microorganisme contenu dans ledit échantillon, ledit tampon de dilution comprenant des polysorbates à une concentration de pas moins de 0,5%, et ledit microorganisme est une bifidobacterium.

2. Utilisation du tampon de dilution pour suspension selon la revendication 1, dans lequel une concentration desdits polysorbates est dans une plage allant de 0,5 à 30%.

3. Utilisation du tampon de dilution pour suspension selon la revendication 1 ou la revendication 2, dans lequel lesdits polysorbates sont au moins un polysorbate choisi dans le groupe constitué du Tween 40, du Tween 60 et du Tween 80.

4. Méthode d'énumération pour un microorganisme contenu dans un échantillon, ladite méthode comprenant :
une étape de préparation d'un tampon de suspension dudit échantillon en utilisant le tampon de dilution pour suspension selon l'une quelconque des revendications 1 à 3, et
une étape de mesure d'un nombre de cellules viables dudit microorganisme contenu dans ledit tampon de suspension, dans laquelle
ledit microorganisme est une bifidobacterium.

5. Méthode d'énumération selon la revendication 4, dans laquelle ledit échantillon est sous une forme poudreuse.

6. Méthode d'énumération selon la revendication 5, dans laquelle ledit échantillon est une formule poudreuse pour nourrissons ou enfants.
